(19) Europäisches Patentamt
European Patent Office
Office européen des brevets

(11) **EP 3 763 413 A1**

(12) **EUROPÄISCHE PATENTANMELDUNG**

(43) Veröffentlichungstag:
**13.01.2021 Patentblatt 2021/02**

(51) Int Cl.:
***A61M 25/10*** *(2013.01)*

(21) Anmeldenummer: **19185653.3**

(22) Anmeldetag: **11.07.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **BIOTRONIK AG**
**8180 Bülach (CH)**

(72) Erfinder: **Fargahi, Amir**
**8180 Bülach (CH)**

(74) Vertreter: **Galander, Marcus**
**Biotronik Corporate Services SE**
**Corporate Intellectual Property**
**Sieversufer 7 - 9**
**12359 Berlin (DE)**

(54) **BALLONKATHETER MIT EINEM EXPANDIERBAREN BALLON SOWIE EINEM SENSOR ZUM MESSEN EINES DURCHMESSERS DES BALLONS**

(57)    Ballonkatheter (1), mit einem Ballon (3), der einen Balloninnenraum (3a) umgibt, der mit einem fluiden Inflationsmedium (M) befüllbar ist, einem Katheterschaft (7), wobei der Ballon (3) mit einem distalen Ende (4) des Katheterschafts (7) verbunden ist, und wobei der Katheterschaft (7) ein mit dem Balloninnenraum (3a) verbundenes Lumen (7a) umgibt, über das das Inflationsmedium (M) in den Balloninnenraum (3a) einleitbar ist, und einem Sensor (9) zum Ermitteln eines momentanen Ballondurchmessers (D). Der Sensor (9) weist zumindest eine erste und eine zweite Elektrode (5, 6), wobei die beiden Elektroden (5, 6) im Balloninnenraum (3a) des Ballons (3) an einer Innenseite (3b) des Ballons (3) angeordnet sind, wobei der Balloninnenraum (3a) mit einem elektrisch leitfähigen Inflationsmedium (M) befüllbar ist, so dass mittels der Elektroden (5, 6) ein elektrischer Widerstand einer zwischen den beiden Elektroden (5, 6) angeordneten Zelle (V) des Inflationsmediums (M) messbar ist.

FIG. 5A

## Beschreibung

Die Erfindung betrifft einen Ballonkatheter sowie ein Verfahren zur Herstellung eines solchen Ballonkatheters.

Liegt der Katheter bei einem Eingriff an der gewünschten Stelle im Blutgefäß, kann der Ballon des Ballonkatheters in der Regel unter hohem Druck (bis zu 16 atm) mit einem Fluid, das ein Kontrastmittel enthält, gefüllt und entsprechend expandiert werden. Durch den starken Druck, den der Ballon auf die Gefäßwand ausübt, werden die Kalkablagerungen bei einer Angioplastie in die Gefäßwand gedrückt, und der Gefäßdurchmesser vergrößert sich. Bei einem derartigen Eingriff ist es natürlich wünschenswert, dass der behandelnde Arzt den beim Expandieren des Ballons sich einstellenden Ballondurchmesser möglichst präzise überwachen kann.

Zur Bestimmung bzw. Abschätzung des momentanen Durchmessers eines Ballons eines Ballonkatheters ist es im Stand der Technik z.B. bekannt, den üblicherweise auf einer sogenannten Compliance Chart des Ballonkatheters gezeigten Zusammenhang zwischen einen am Ballonkatheter anliegenden bzw. einen im Balloninnenraum herrschenden Inflationsdruck und dem Durchmesser des Ballons zu verwenden. Der Inflationsdruck ist in der Regel messbar, so dass hieraus der entsprechende Durchmesser des Ballons abschätzbar ist.

Weiterhin sind Ballonkatheter bekannt, deren Ballons Sensoren auf der Ballonoberfläche aufweisen. Derartige Sensoren müssen eine multiaxiale Dehnung von bis zu 30% bestehen können. Ebenfalls wichtig ist eine Wiederholbarkeit der Messung, welche jedoch in der Regel durch die viskoelastische Eigenschaft des polymeren Ballons nicht gegeben ist.

Weiterhin besteht das Risiko einer Rissbildung in den Sensoren bei mechanischer Belastung. Eine weitere Problematik ist der Einsatz von Tinten mit Nanopartikeln als Leitermaterial und die damit verbundene Problematik der Biokompatibilität und der Blutverträglichkeit (Hämokompatibilität) solcher Sensoren.

Schließlich zeigen auf der Ballonoberfläche angeordnete Sensoren ebenfalls Probleme beim Falten des Ballons und auch beim Einsatz im Patientenkörper. Insbesondere können auf der Ballonoberfläche angeordnete Sensoren durch die vergleichsweise harten und abrasiv wirkenden Stenosen beeinträchtigt werden.

Die WO97/142871 beschreibt einen Katheter mit einem Ballon, der elektrische Widerstände aus einem Polymer oder einem Elastomer aufweist, die zum Messen eines Gefäßdurchmessers verwendet werden.

Weiterhin offenbart die US 5,827,273 eine Ablationsvorrichtung, bei der eine elektrolytische Lösung einen Ballon der Vorrichtung füllt und durch Öffnungen des Ballons aus dem Ballon ausströmen kann.

Hiervon ausgehend liegt der vorliegenden Erfindung die Aufgabe zugrunde, einen Ballonkatheter bereitzustellen, der ein einfaches und praktikables Design aufweist und es ermöglicht einen Ballondurchmesser insbesondere kontinuierlich zu messen, wobei insbesondere die Biokompatibilität des Ballonkatheters gewährleistet werden soll.

Diese Aufgabe wird durch einen Ballonkatheter mit den Merkmalen des Anspruchs 1 gelöst. Vorteilhafte Ausgestaltungen des Ballonkatheters sind in den entsprechenden Unteransprüchen angegeben und werden nachfolgend beschrieben.

Gemäß Anspruch 1 wird ein Ballonkatheter offenbart, mit:

- einem in einer axialen Richtung erstreckten Ballon, der einen Balloninnenraum umgibt, der zur Expansion des Ballons in einer radialen Richtung des Ballons mit einem fluiden Inflationsmedium befüllbar ist,
- einem in der axialen Richtung erstreckten Katheterschaft, wobei der Ballon mit einem distalen Ende des Katheterschafts verbunden ist, und wobei der Katheterschaft ein mit dem Balloninnenraum verbundenes Lumen aufweist, über das das Inflationsmedium in den Balloninnenraum einleitbar ist, und
- einem Sensor zum Ermitteln eines momentanen Ballondurchmessers des Ballons in der radialen Richtung.

Erfindungsgemäß ist vorgesehen, dass der Sensor zum Ermitteln des momentanen Ballondurchmessers zumindest eine erste und eine zweite Elektrode aufweist, wobei die beiden Elektroden im Balloninnenraum des Ballons an einer dem Balloninnenraum zugewandten Innenseite des Ballons angeordnet sind, wobei der Balloninnenraum mit einem elektrisch leitfähigen Inflationsmedium befüllbar ist, so dass mittels der Elektroden ein elektrischer Widerstand einer zwischen den beiden Elektroden angeordneten Zelle des Inflationsmediums messbar ist.

Das Inflationsmedium kann in einem geeigneten, mit dem Lumen des Katheterschaft verbundenen oder verbindbaren Reservoir gespeichert sein und kann zusammen mit dem Reservoir eine Komponente des Ballonkatheters bilden.

Im Rahmen der vorliegenden Erfindung werden Komponenten oder Orte, die entlang des Ballonkatheters näher am Anwender (Arzt) des Ballonkatheters liegen als proximal bezeichnet. Entsprechend werden Komponenten oder Orte, die weiter vom Anwender des Ballonkatheters entfernt sind als distal bezeichnet.

Mittels der beiden auf der Innenseite des Ballons angeordneten Elektroden ist also eine elektrische Widerstandmessung durchführbar. Diese Messung kann fortlaufend vorgenommen werden. Während der Expansion bzw. Deflation des Ballons wird der Abstand zwischen den beiden Elektroden variieren. Die Widerstandsänderung korreliert mit der Durchmesseränderung des Ballons. Die Leitfähigkeit und die Oberflächen der Elektroden bleiben unverändert.

Weiterhin ist aufgrund der elektrodenbasierten Messung mit Vorteil eine Biokompatibilität gegeben bzw. leicht realisierbar,, da das biologische Gewebe weiterhin nur über erprobte Ballonmaterialien (die Ballonaußenseite) kontaktiert wird. Weiterhin ist aufgrund der Elektrodenanordnung die Funktionalität der Durchmesserbestimmung trotz einer bi-axialen Dehnung des Ballons sichergestellt.

Gemäß einer Ausführungsform des Ballonkatheters ist vorgesehen, dass der Sensor eine Auswertungseinheit aufweist, die elektrisch leitend mit den beiden Elektroden verbunden ist oder mit diesen verbindbar ist und dazu konfiguriert ist, aus dem elektrischen Widerstand der Zelle des Inflationsmediums den Durchmesser des Ballons zu berechnen.

Weiterhin ist gemäß einer Ausführungsform des Ballonkatheters vorgesehen, dass der Ballon einen zylindrischen Abschnitt aufweist, wobei die beiden Elektroden im zylindrischen Abschnitt des Ballons an der Innenseite des Ballons angeordnet sind.

Weiterhin ist gemäß einer Ausführungsform des Ballonkatheters vorgesehen, dass die beiden Elektroden in der radialen Richtung des Ballons einander gegenüberliegen. Beim Expandieren des Ballons entfernen sich die Elektroden voneinander, so dass sich der elektrische Widerstand entsprechend erhöht. Hieraus lässt sich automatisch mittels der Auswertungseinheit der momentane Durchmesser des Ballons (z.B. während des Expandierens des Ballons) kontinuierlich messen. Weiterhin kann die Auswerteinheit dazu ausgebildet sein, die Messdaten des Sensors bzw. die errechneten Durchmesser zu speichern und/oder an eine externe Datenbank zu übermitteln.

Weiterhin ist gemäß einer Ausführungsform des Ballonkatheters vorgesehen, dass die beiden Elektroden jeweils röntgensichtbar sind (z.B. indem die beiden Elektroden goldbeschichtet sind).

Weiterhin ist gemäß einer Ausführungsform des Ballonkatheters vorgesehen, dass die erste Elektrode mit einem ersten elektrischen Leiter verbunden ist, und dass die zweite Elektrode mit einem zweiten elektrischen Leiter verbunden ist, wobei sich die beiden Leiter ausgehend von der jeweiligen Elektrode entlang des Katheterschafts erstrecken.

Über diese Leiter kann die jeweilige Elektrode mit der Auswertungseinheit verbunden sein bzw. werden.

Weiterhin ist gemäß einer Ausführungsform des Ballonkatheters vorgesehen, dass der erste Leiter aus einer Formgedächtnislegierung gebildet ist, wobei der erste Leiter einen distalen Abschnitt aufweist, der über ein distales Ende mit der ersten Elektrode und über ein proximales Ende mit einem proximalen Abschnitt des ersten Leiters verbunden ist, wobei sich der distale Abschnitt des ersten Leiters oberhalb einer Umwandlungstemperatur der Formgedächtnislegierung in radialer Richtung des Ballons nach außen erstreckt.

In analoger Weist ist bevorzugt auch der zweite Leiter aus einer Formgedächtnislegierung gebildet, wobei der zweite Leiter einen distalen Abschnitt aufweist, der über ein distales Ende mit der zweiten Elektrode und über ein proximales Ende mit einem proximalen Abschnitt des zweiten Leiters verbunden ist, wobei sich der distale Abschnitt des zweiten Leiters oberhalb einer Umwandlungstemperatur der Formgedächtnislegierung des zweiten Leiters in radialer Richtung des Ballons nach außen erstreckt.

Die beiden distalen Abschnitte können hierbei jeweils bezüglich des entsprechenden proximalen Abschnitts einen abgewinkelten und/oder radial nach außen gekrümmten Verlauf aufweisen. Insbesondere erstrecken sich die beiden distalen Abschnitte oberhalb der Umwandlungstemperatur in entgegengesetzte radiale Richtungen nach außen zur Innenseite des Ballons hin bzw. zur jeweiligen Elektrode hin.

Weiterhin ist gemäß einer Ausführungsform des Ballonkatheters vorgesehen, dass der distale Abschnitt des ersten Leiters sich unterhalb der Umwandlungstemperatur der Formgedächtnislegierung des ersten Leiters parallel zum proximalen Abschnitt des ersten Leiters erstreckt. In analoger Weise ist vorzugsweise vorgesehen, dass sich der distale Abschnitt des zweiten Leiters unterhalb der Umwandlungstemperatur der Formgedächtnislegierung des zweiten Leiters parallel zum proximalen Abschnitt des zweiten Leiters erstreckt. Dies ermöglicht eine kompakte Form des Ballons beim Vorschieben des Ballons zum Zielort in einem Gefäß eines Patienten (vor dem Expandieren des Ballons). Die besagte parallele Erstreckung der Abschnitte bezieht sich natürlich auf eine entsprechend lineare Anordnung/Erstreckung des Ballons und des flexiblen Katheterschafts.

Die Formgedächtnislegierungen der beiden Leiter sind vorzugsweise gleich. Als Formgedächtnislegierung kann z.B. eine geeignete Nickel-Titan-Legierung verwendet werden, wie z.B. Nitinol. Die jeweilige Umwandlungstemperatur liegt vorzugsweise im Bereich von 15°C bis 35°C.

Weiterhin ist gemäß einer Ausführungsform des Ballonkatheters vorgesehen, dass die beiden Elektroden jeweils plattenförmig ausgebildet sind. Die Elektroden können z.B. an den jeweiligen Leiter angefügt sein. Es ist auch möglich, ein distales Ende des jeweiligen Leiters z.B. plattenförmig umzuformen. Die Elektroden können in beiden Fällen mit einem Metall wie z.B. Gold beschichtet sein. Sofern die Elektroden an den jeweiligen Leiter angefügt sind, können Sie auch aus einem anderen geeigneten Metall, insbesondere Gold bzw. einer Goldlegierung, gebildet sein.

Weiterhin ist gemäß einer Ausführungsform des Ballonkatheters vorgesehen, dass die beiden Leiter jeweils mit einer elektrischen Isolierung ummantelt sind (z.B. aus PUR), so dass im Balloninnenraum lediglich die Elektroden freiliegen und das Inflationsmedium kontaktieren können.

Weiterhin ist gemäß einer Ausführungsform des Ballonkatheters vorgesehen, dass in dem Lumen des Katheterschafts ein Innenschaft zur Aufnahme eines Führungsdrahts angeordnet ist, wobei die beiden Leiter auf einer Außenseite des Innenschafts im Lumen des Katheterschafts verlaufen bzw. dort fixiert sein können. Der Innenschaft kann koaxial zum Katheterschaft angeordnet sein.

Gemäß einer alternativen Ausführungsform des Ballonkatheters kann vorgesehen sein, dass die beiden Leiter in dem Lumen des Katheterschafts angeordnet sind, wobei der Katheterschaft zumindest ein weiteres Lumen zur Aufnahme eines Führungsdrahts aufweist. Der Katheterschaft kann dabei noch weitere Lumen aufweisen. Bei einer solchen Variante des Ballonkatheters weist der Katheterschaft also separate und nebeneinander verlaufende Lumen auf.

Gemäß einer weiteren Ausführungsform der Erfindung ist vorgesehen, dass der Ballonkatheter mehrere aus einer ersten und einer zweiten Elektrode gebildete Elektrodenpaare aufweist, mit denen jeweils ein elektrischer Widerstand einer Zelle des Inflationsmediums messbar ist, das zwischen dem betreffenden Elektrodenpaar angeordnet ist. Hierdurch kann die Genauigkeit der Durchmesserberechnung gesteigert werden. Insbesondere können entlang der axialen Richtung des Ballons mehrere momentane Durchmesser des Ballons erfasst werden, was auch Rückschlüsse auf die 3D-Form des Ballons beim Expandieren zulässt.

Ein weiterer Aspekt der vorliegenden Erfindung betrifft ein Verfahren zum Messen eines Ballondurchmessers unter Verwendung eines erfindungsgemäßen Ballonkatheters, aufweisend die Schritte:

- Expandieren des Ballons in der radialen Richtung durch Einleiten eines fluiden und elektrisch leitfähigen Inflationsmediums in den Balloninnenraum, so dass zwischen den beiden Elektroden eine Zelle des Inflationsmediums gebildet wird, und
- Messen eines elektrischen Widerstands der Zelle des Inflationsmediums mittels der Elektroden und automatisches Berechnen des Durchmessers des Ballons anhand des gemessenen elektrischen Widerstands.

Schließlich betrifft ein weiterer Aspekt der vorliegenden Erfindung ein Verfahren zur Herstellung einer Elektrode für einen Ballonkatheter, insbesondere für einen erfindungsgemäßen Ballonkatheter, aufweisend die Schritte:

- Bereitstellen eines Drahts aus einer Formgedächtnislegierung, wobei der Draht einen distalen und einen damit verbundenen proximalen Abschnitt aufweist,
- Umformen und Wärmebehandeln des distalen Abschnitts derart, dass der distale Abschnitt bei einer Temperatur oberhalb einer Umwandlungstemperatur einen gekrümmten und/oder abgewinkelten Verlauf gegenüber dem proximalen Abschnitt aufweist,
- Ausbilden einer plattenförmigen Elektrode an einem distalen Ende des distalen Abschnitts (z.B. durch Beschichten des distalen Endes mit einem geeigneten Metall, wie z.B. Gold, oder durch Anfügen eines plattenförmigen Elektrodenelements, das z.B. aus Gold bzw. einer Goldlegierung gebildet ist), und
- Ummanteln des distalen und des proximalen Abschnitts des Drahts mit einer elektrischen Isolierung (z.B. aus PUR), wobei die Elektrode und insbesondere auch ein proximales Ende des Drahts freigelassen wird.

Dieses Verfahren kann auch im Rahmen eines Verfahrens zur Herstellung eines Ballonkatheters, insbesondere eines erfindungsgemäßen Ballonkatheters, verwendet werden, indem die Elektroden zweier solchermaßen hergestellter Drähte an der Innenseite des Ballons des Ballonkatheters festgelegt werden und die mit den Elektroden verbundenen Leiter bzw. Drähte jeweils in einem Lumen des Katheterschafts angeordnet werden (oder in einer sonstigen Weise geeignet verlegt werden). Über die Leiter bzw. Drähte kann schließlich die Auswertungseinheit an die Elektroden angeschlossen werden. Der im erfindungsgemäßen Verfahren verwendete Schritt des Umformen und Wärmebehandelns zum Einprägen der Form ist dem Fachmann bekannt. Hierzu wird bevorzugt der distale Abschnitt unter Formzwang auf eine Temperatur im Bereich zwischen 450°C und 550°C erwärmt und anschließend schnell abgekühlt. Die genaue Parameter hängen von Legierung und gewünschten Verformungsgrad ab und bilden Teil des fachmännischen Könnens.

Im Folgenden sollen Ausführungsformen der Erfindung sowie weitere Merkmale und Vorteile der Erfindung anhand der Figuren erläutert werden. Es zeigen:

Fig. 1        eine schematische Darstellung des Messprinzips des Sensors einer erfindungsgemäßen Ballonkatheters;

Fig. 2A - 2D     eine schematische Darstellung der möglichen Schritte zur Herstellung einer Elektrode für einen Sensor eines erfindungsgemäßen Ballonkatheters;

Fig. 3        eine schematische Darstellung der Anordnung der beiden Elektroden des Sensors einer Ausführungsform des erfindungsgemäßen Ballonkatheters;

Fig. 4A - 4C     schematische Schnittdarstellungen eines Ballons sowie der an der Innenseite des Ballons angeordneten Elektroden einer Ausführungsform eines erfindungsgemäßen Ballonkatheters;

Fig. 5A        eine schematische Darstellung einer Ausführungsform eines erfindungsgemäßen Ballonkatheters;

Fig. 5B eine schematische Schnittdarstellung der Ausführungsform nach Fig. 5A; und

Fig. 5C eine schematische Schnittdarstellung einer alternativen Ausführungsform eines erfindungsgemäßen Ballonkatheters.

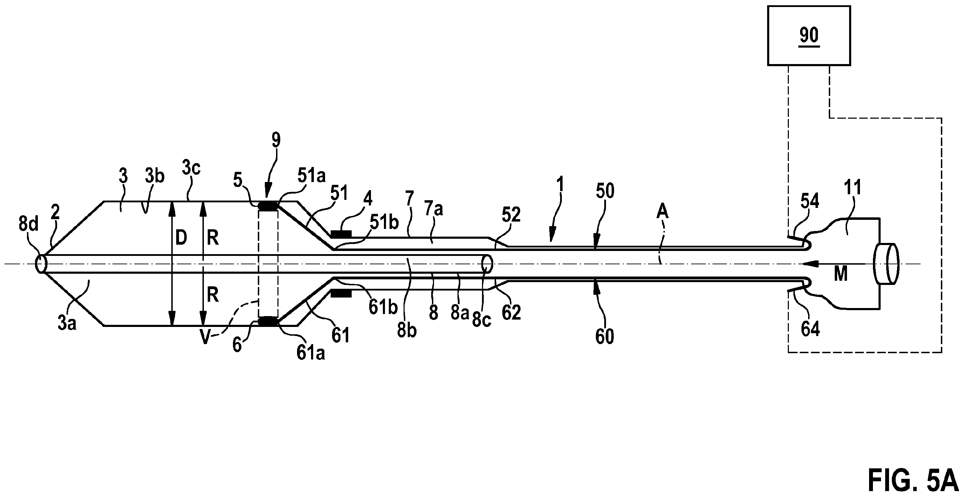

Die Erfindung betrifft, wie z.B. anhand der Ausführungsform gemäß der Fig. 5A ersichtlich ist, einen Ballonkatheter 1, der einen in einer axialen Richtung z erstreckten Ballon 3 aufweist, der einen Balloninnenraum 3a umgibt, der zur Expansion des Ballons 3 in einer radialen Richtung R des Ballons 3 mit einem fluiden Inflationsmedium M befüllbar ist. Die jeweilige radiale Richtung R steht senkrecht auf der axialen Richtung z und weist nach außen zu einer Innenseite 3b des Ballons 3 hin. Weiterhin weist der Ballonkatheter 1 einem in der axialen Richtung z erstreckten Katheterschaft 7 auf, wobei der Ballon 3 mit einem distalen Ende 4 des Katheterschafts 7 verbunden ist, und wobei der Katheterschaft 7 ein mit dem Balloninnenraum 3a verbundenes Lumen 7a umgibt, über das das Inflationsmedium M in den Balloninnenraum 3a einleitbar ist. Ferner weist der Ballonkatheter 1 einen Sensor 9 zum Ermitteln eines momentanen Ballondurchmessers D des Ballons 3 bezüglich der radialen Richtung R des Ballons auf. Der Ballon 3 ist vorzugsweise zylindersymmetrisch bezüglich der axialen Richtung z ausgebildet.

Erfindungsgemäß ist vorgesehen, dass der Sensor 9 zum Ermitteln des momentanen Ballondurchmessers D zumindest eine erste und eine zweite Elektrode 5, 6 aufweist, wobei die beiden Elektroden 5, 6 im Balloninnenraum 3a des Ballons 3 an der Innenseite 3b des Ballons 3 angeordnet sind. Da der Balloninnenraum 3a mit einem elektrisch leitfähigen Inflationsmedium M befüllbar ist, ist nunmehr mittels der Elektroden 5, 6 ein elektrischer Widerstand einer zwischen den beiden Elektroden 5, 6 angeordneten Zelle V des Inflationsmediums M messbar.

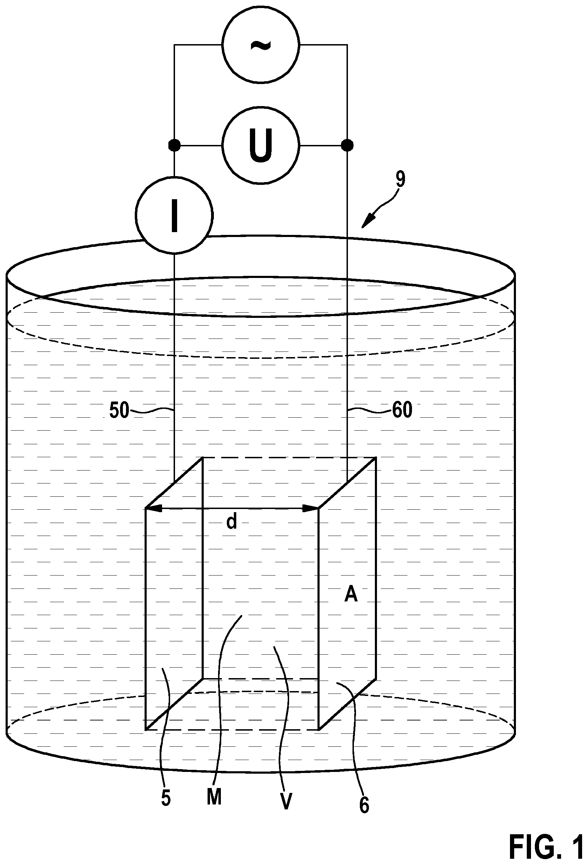

Dies ist in der Fig. 1 schematisch dargestellt. Hierbei ergibt sich der elektrische Widerstand $R_Z$ der besagten Inflationsmediumszelle V zu:

$$R_z = \left(\frac{d}{A}\right) \cdot \left(\frac{1}{\sigma}\left[\text{AF1}\right]\right),$$

wobei d der Abstand zwischen den beiden Elektroden 5, 6 ist, und wobei A die Fläche der jeweiligen Elektrode 5, 6 ist, und wobei $\sigma$ die Leitfähigkeit des fluiden Inflationsmediums ist M. So kann beispielsweise durch Messung der Spannung U und des Stroms I mittels einer Auwertungseinheit der Widerstand $R_Z$ berechnet werden, woraus der Elektrodenabstand d berechnet werden kann. Aufgrund der Kenntnisse über die Geometrie des Ballons 3 kann aus dem Elektrodenabstand d der eigentlichen Ballondurchmesser D berechnet werden.

Zur Inflation bzw. zum Expandieren des Ballons 3 können z.B. Salin-Lösungen (0.9%) oder Kontrastmittel (z.B. Hexabrix 320 oder Visipaque) M verwendet werden. Diese Flüssigkeiten bzw. fluiden Inflationsmittel M sind elektrisch leitende Materialien. Die elektrische Leitfähigkeit von Salzwasser beträgt in etwa 5 S/m bis 10 S/m.

Da die beiden Elektroden 5, 6 auf der Innenseite 3b des Ballons 3 einander gegenüberliegend angeordnet sind, vorzugsweise im zylindrischen Bereich 3c des Ballons 3, ist eine Widerstandmessung in der oben beschriebenen Weise durchführbar. Während der Inflation bzw. Deflation des Ballons 3 wird der Abstand d zwischen den beiden Elektroden 5,6 variieren. Diese Widerstandsänderung korreliert mit der eigentlichen Durchmesseränderung des Ballons 3.

Die beiden Elektroden 5, 6 sind gemäß Fig. 5A vorzugsweise über je einen elektrischen Leiter 50, 60 mit einer Auswertungseinheit 90 des Sensors 9 verbunden, die anhand des gemessenen Widerstandswerts sowie einer geeigneten Kalibrierung automatisch den momentanen Durchmesser D des Ballons 3 in der radialen Richtung R bestimmt.

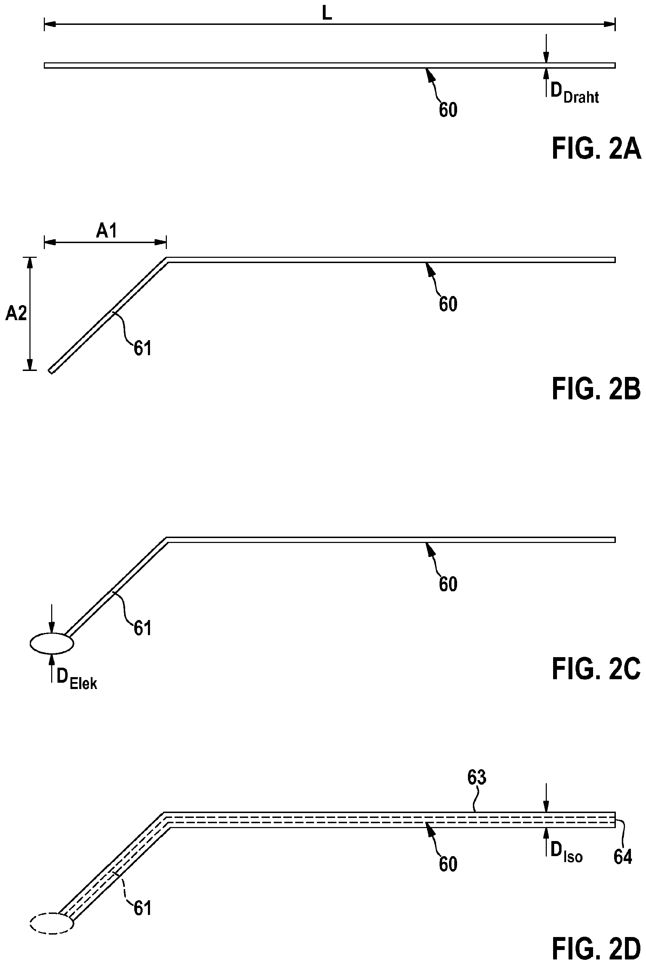

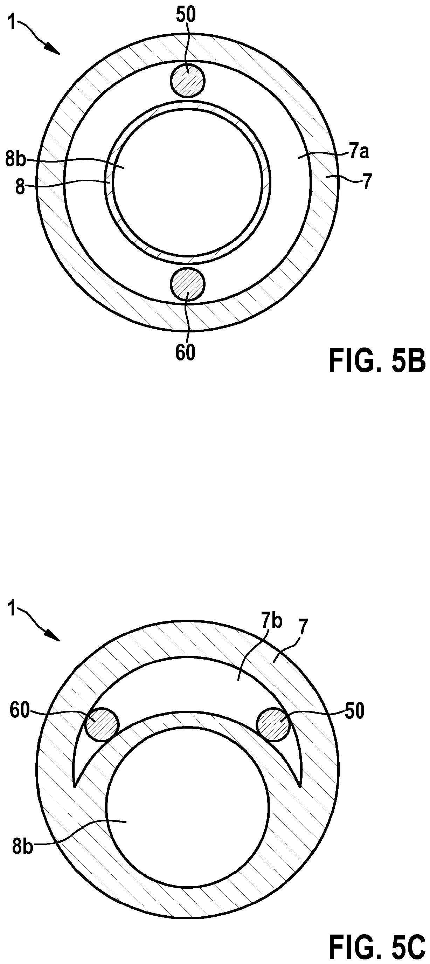

Die beiden elektrischen Leiter 50, 60, über die die im Balloninnenraum 3a angeordneten Elektroden 5, 6 des Sensors 9 mit der Auswertungseinheit 90 verbunden sind bzw. verbindbar sind, können z.B. nach Art der Figuren 2A bis 3 ausgestaltet sein. Die beiden Leiter 50, 60 können dabei ausgehend von der jeweiligen Elektrode 5 bzw. 6 gemäß Fig. 5A entlang des Katheterschafts 7 verlaufen. Je nach Kathetertyp können die beiden Leiter 50, 60 z.B. gemäß Fig. 5A in einem Lumen 7a des Ballonkatheters 1 verlaufen, über das das Inflationsmedium M ausgehend vom proximalen Einlass 11 des Ballonkatheters 1 in den Balloninnenraum 3a einleitbar ist. Hierbei kann in dem Lumen 7a ein Innenschaft 8 angeordnet sein, der seinerseits eine Lumen 8b zur Aufnahme eines nicht gezeigten Führungsdrahts aufweisen kann. Die beiden Leiter 50, 60 können dabei auf einer Außenseite 8a des Innenschafts 8 angeordnet sein. Der Innenschaft 8 kann eine laterale Öffnung 8c aufweisen, die einen Ausgang für den Führungsdraht bildet, wobei an der distalen Spitze 2 des Ballonkatheters 1 vorzugsweise eine weitere Öffnung 8d des Innenschaftlumens 8b vorgesehen ist, die einen Eingang für den Führungsdraht in das Innenschaftlumen 8b bildet. Fig. 5B zeigt im Querschnitt die koaxiale Anordnung von Innenschaft 8 und Katheterschaft 7 mit den entsprechenden Lumen 8b für den Führungsdraht und dem Lumen 7a für das Inflationsmedium und den darin angeordneten Leitern 50, 60. Fig. 5C zeigt eine alternative Ausführungsform mit einer parallelen Anordnung des Lumens 8b für den Führungsdraht und des Lumens 7b für das Inflationsmedium.

Die erfindungsgemäße Lösung ist natürlich auf alle erdenklichen Ballonkathetertypen, die einen mit einem fluiden Medium expandierbaren Ballon aufweisen, anwendbar. Fig. 5B zeigt eine alternative Variante in einer Schnittansicht senkrecht zur axialen Richtung z, wobei hier der Katheterschaft 7 separate, nebeneinander angeordnete Lumen 7a und 8b aufweist. Das Lumen 7a dient analog zum Führen des Inflationsmediums M und nimmt gleichzeitig die Leiter 50, 60 auf. Das parallele Lumen 8b dient analog zur Aufnahme eines Führungsdrahts.

Die beiden Leiter 50, 60 können z.B. mit je einem Ende 54, 64 am proximalen Ende des Katheterschafts 7 aus dem Ballonkatheter 1 herausgeführt sein und können ferner über eine geeignete elektrische Leitungsverbindung mit der Auswertungseinheit 90 verbunden sein. Es ist auch denkbar, die Sensormesswerte des Sensors 9 vom proximalen Ende des Ballonkatheters aus über eine kabellose Signalverbindung an die Auswerteeinheit zu übermitteln.

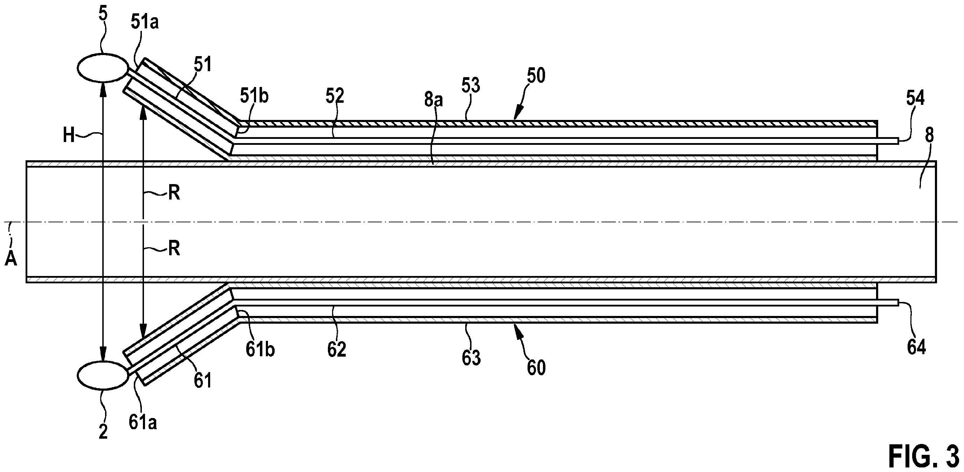

Vorzugsweise sind die beiden Leiter 50, 60 aus einer Formgedächtnislegierung gebildet, wobei die beiden Leiter 50, 60 jeweils einen distalen Abschnitt 51, 61 aufweisen, der über ein distales Ende 51a, 61a mit der ersten Elektrode 5 bzw. mit der zweiten Elektrode 6 und über ein proximales Ende 51b, 61b mit einem proximalen Abschnitt 52, 62 des ersten Leiters 50 bzw. des zweiten Leiters 60 verbunden ist, wobei sich der jeweilige distale Abschnitt 51, 61 oberhalb einer Umwandlungstemperatur der Formgedächtnislegierung in radialer Richtung R des Ballons 3 nach außen erstreckt und dabei insbesondere abgewinkelt zum zugeordneten proximalen Abschnitt 52, 62 verläuft (vgl. Fig. 3). Unterhalb der Umwandlungstemperatur sind die Abschnitte 51, 52 bzw. 61, 62 der Leiter 50, 60 vorzugsweise linear erstreckt. Die Elektroden 5, 6 sind vorzugsweise als plattenförmige Elemente an die distalen Enden 51a, 61a der distalen Abschnitte 51, 61 angeschlossen. Weiterhin sind die Leiter 50, 60 vorzugsweise mit einer elektrischen Isolierung 53, 63 ummantelt.

Die Figuren 2A bis 2D zeigen Schritte einer Ausführungsform eines Herstellungsverfahrens für die Elektroden 5, 6, das hier anhand der zweiten Elektrode 6 gezeigt wird und für die erste Elektrode 5 analog durchführbar ist.

Hierzu wird gemäß Fig. 2A vorzugsweise ein Draht 60 aus einer geeigneten Nickel-Titan-Legierung verwendet (z.B. Nitinol). Der Drahtdurchmesser $D_{Draht}$ kann z.B. 50 μm bis 100 μm betragen, bei einer Länge L von z.B. L=1500mm (vgl. Fig. 2A). Die Legierung kann z.B. nach ASTM 2063 spezifiziert werden, wobei die kritische Umwandlungstemperatur (Af) vorzugsweise zwischen 15°C und 35°C liegt. Der spezifische elektrische Widerstand liegt z.B. bei 76μ Ωcm für ASTM F2063 Nitinol.

Der Nitinol-Draht 60 wird weiterhin gemäß Figur 2B umgeformt und wärmebehandelt, um eine Abwinklung des distalen Abschnitts 61 zu erhalten, die sich oberhalb der Umwandlungstemperatur einstellt. Die Längen bzw. Dimensionen A1 und A2 können z.B. A1 =3mm und A2=3mm betragen.

Zur Erzeugung der Elektrode 6 kann der Nitinol-Draht 60 am distalen Ende 61a des distalen Abschnitts 67 gemäß Figur 2C mit Gold beschichtet werden. Alternativ kann z.B. ein plattenförmiges Elektrodenelement 6 als Elektrode 6 am distalen Ende 61a festgelegt werden, z.B. durch Löten oder Schweißen. Der Durchmesser $D_{Elek}$ der Elektrode 6 kann z.B. 10 μm betragen.

Schließlich erhält der Draht bzw. Leiter 60 eine elektrische Isolierung 63 aus einem dielektrischen Material, z.B. PUR. Die Dicke $D_{Iso}$ der Isolierung kann z.B. 5 μm bis 10 μm betragen.

Die solchermaßen hergestellten Elektroden 5, 6 können dann im Rahmen einer Vormontage auf einem distalen Bereich bzw. einer Außenseite 8a des Innenschaftes 8 des Ballonkatheters 1 fixiert werden (vgl. Fig. 3). Der Abstand H der Elektroden ist dabei in etwa so groß wie der Balloninnendurchmesser und variiert während der Inflation bzw. Deflation des Ballons 3. Die proximalen Enden 54, 64 der Leiter 50, 60 werden nicht isoliert, so dass hieran weitere Leiter oder Anschlüsse elektrisch angebunden werden können.

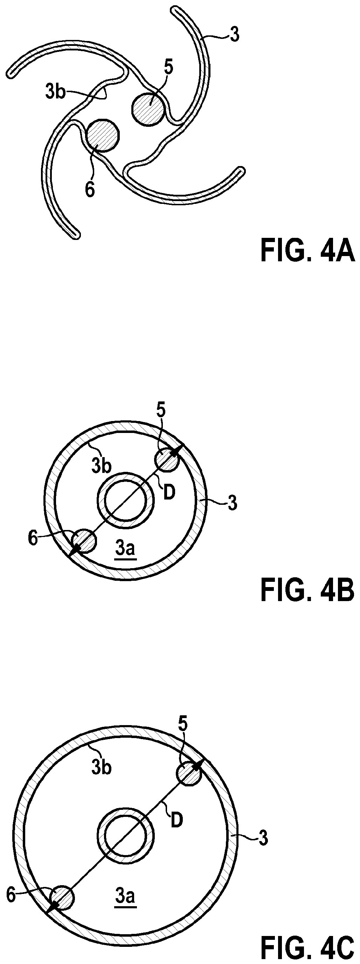

Bei einem noch nicht expandierten Ballon 3 liegen die an der Balloninnenseite 3b festgelegten Elektroden 5, 6 dicht aneinander (vgl. Fig. 4A). Während der Expansion des Ballons 3 durch Einleiten des Inflationsmediums M in den Balloninnenraum 3a vergrößert sich der Ballondurchmesser D entsprechend der Fig. 4B (bei Nominaldruck) bzw. Fig. 4C (bei spezifizierten Maximaldruck). Insbesondere aus den Figuren 4B und 4C wird ersichtlich, dass der Abstand der Elektroden 5, 6 zueinander aufgrund der geringen Ballon- und Elektrodenstärken im Wesentlichen dem Ballondurchmesser D entspricht.

Die Erfindung erlaubt mit Vorteil eine präzise Messung des Ballondurchmessers, die unabhängig von bi-axialen bzw. multiaxialen Dehnungen des Ballons funktioniert. Weiterhin kann die Messung des Durchmessers grundsätzlich im Balloninnenraum durchgeführt werden, d.h., die vorliegende Methode ist geeignet für Ballonkatheter, deren Ballon einen auf dem Ballon angeordnetes Implantat, beispielsweise einen Stent oder eine stentbasierte Herzklappenprothese, aufweiten soll. Gleichermaßen ist sie für Ballonkatheter zur Durchführung einer Ballon-Angioplastie geeignet. Weiterhin kann im Rahmen der vorliegenden Erfindung die Ballondilatation mit preisgünstigen Salin-Lösungen durchgeführt werden. Die vorliegende Erfindung realisiert ferner eine robuste Messmethode, da das Risiko einer Verletzung der Elektroden gering ist. Schließlich besteht kein Biokompatibilitätsrisiko und die Elektroden können mit Vorteil röntgensichtbar gestaltet werden.

**Patentansprüche**

1. Ballonkatheter (1), mit:

    - einem in einer axialen Richtung (z) erstreckten Ballon (3), der einen Balloninnenraum (3a) umgibt, der zur Expansion des Ballons (3) in einer radialen Richtung (R) des Ballons (3) mit einem fluiden Inflationsmedium (M) befüllbar ist,
- einem in der axialen Richtung (z) erstreckten Katheterschaft (7), wobei der Ballon (3) mit einem distalen Ende (4) des Katheterschafts (7) verbunden ist, und wobei der Katheterschaft (7) ein mit dem Balloninnenraum (3a) verbundenes Lumen (7a) umgibt, über das das Inflationsmedium (M) in den Balloninnenraum (3a) einleitbar ist, und
- einem Sensor (9) zum Ermitteln eines momentanen Ballondurchmessers (D) des Ballons (3) in der radialen Richtung (R),

**dadurch gekennzeichnet,**

**dass** der Sensor (9) zum Ermitteln des momentanen Ballondurchmessers (D) zumindest eine erste und eine zweite Elektrode (5, 6) aufweist, wobei die beiden Elektroden (5, 6) im Balloninnenraum (3a) des Ballons (3) an einer Innenseite (3b) des Ballons (3) angeordnet sind, wobei der Balloninnenraum (3a) mit einem elektrisch leitfähigen Inflationsmedium (M) befüllbar ist, so dass mittels der Elektroden (5, 6) ein elektrischer Widerstand einer zwischen den beiden Elektroden (5, 6) angeordneten Zelle (V) des Inflationsmediums (M) messbar ist.

2. Ballonkatheter nach Anspruch 1, **dadurch gekennzeichnet, dass** der Sensor (9) eine Auswertungseinheit (90) aufweist, die elektrisch leitend mit den beiden Elektroden (5, 6) verbunden ist und dazu konfiguriert ist, aus dem elektrischen Widerstand der Zelle (V) des Inflationsmediums (M) den Durchmesser (D) des Ballons (3) zu berechnen.

3. Ballonkatheter nach Anspruch 1 oder 2, **dadurch gekennzeichnet, dass** der Ballon (3) einen zylindrischen Abschnitt (3c) aufweist, wobei die beiden Elektroden (5, 6) im zylindrischen Abschnitt (3c) des Ballons (3) an der Innenseite (3b) des Ballons (3) angeordnet sind.

4. Ballonkatheter nach einem der vorhergehenden Ansprüche, **dadurch gekennzeichnet, dass** die beiden Elektroden (5, 6) in der radialen Richtung (R) des Ballons (3) einander gegenüberliegen.

5. Ballonkatheter nach einem der vorhergehenden Ansprüche, **dadurch gekennzeichnet, dass** der Sensor (9) dazu ausgebildet ist, den momentanen Durchmesser (D) des Ballons (3) kontinuierlich zu messen.

6. Ballonkatheter nach Anspruch 2 oder einem der Ansprüche 3 bis 5 soweit rückbezogen auf Anspruch 2, **dadurch gekennzeichnet, dass** die Auswerteeinheit (90) dazu ausgebildet ist, Messdaten des Sensors (9) zu speichern und/oder an eine externe Datenbank zu übermitteln.

7. Ballonkatheter nach einem der vorhergehenden Ansprüche, **dadurch gekennzeichnet, dass** die beiden Elektroden (5, 6) jeweils röntgensichtbar sind.

8. Ballonkatheter nach einem der vorhergehenden Ansprüche, **dadurch gekennzeichnet, dass** die erste Elektrode (5) mit einem ersten elektrischen Leiter (50) verbunden ist, und dass die zweite Elektrode (6) mit einem zweiten elektrischen Leiter (60) verbunden ist, wobei sich die beiden Leiter (50, 60) entlang des Katheterschafts (7) erstrecken.

9. Ballonkatheter nach Anspruch 8, **dadurch gekennzeichnet, dass** der erste Leiter (50) aus einer Formgedächtnislegierung gebildet ist, wobei der erste Leiter (50) einen distalen Abschnitt (51) aufweist, der über ein distales Ende (51a) mit der ersten Elektrode (5) und über ein proximales Ende (51b) mit einem proximalen Abschnitt (52) des ersten Leiters (50) verbunden ist, wobei sich der distale Abschnitt (51) des ersten Leiters (50) oberhalb einer Umwandlungstemperatur der Formgedächtnislegierung in radialer Richtung (R) des Ballons (3) nach außen erstreckt; und/oder wobei der zweite Leiter (60) aus einer Formgedächtnislegierung gebildet ist, wobei der zweite Leiter (60) einen distalen Abschnitt (61) aufweist, der über ein distales Ende (61a) mit der zweiten Elektrode (6) und über ein proximales Ende (61b) mit einem proximalen Abschnitt (62) des zweiten Leiters (60) verbunden ist, wobei sich der distale Abschnitt (61) des zweiten Leiters (60) oberhalb einer Umwandlungstemperatur der Formgedächtnislegierung des zweiten Leiters (60) in radialer Richtung (R) des Ballons (3) nach außen erstreckt.

10. Ballonkatheter nach Anspruch 9, **dadurch gekennzeichnet, dass** sich der distale Abschnitt (51) des ersten Leiters (50) unterhalb der Umwandlungstemperatur der Formgedächtnislegierung des ersten Leiters (50) parallel zum proximalen Abschnitt (52) des ersten Leiters (50) erstreckt; und/oder dass sich der distale Abschnitt (61) des zweiten Leiters (60) unterhalb der Umwandlungstemperatur der Formgedächtnislegierung des zweiten Leiters (60) parallel zum proximalen Abschnitt (62) des zweiten Leiters (60) erstreckt.

11. Ballonkatheter nach einem der vorhergehenden Ansprüche, **dadurch gekennzeichnet, dass** die beiden Elektroden (5, 6) jeweils plattenförmig ausgebildet sind.

12. Ballonkatheter nach Anspruch 8 oder nach einem der Ansprüche 9 bis 11 soweit rückbezogen auf Anspruch 8, **dadurch gekennzeichnet, dass** die beiden Leiter (50, 60) jeweils mit einer elektrischen Isolierung (53, 63) ummantelt sind.

**13.** Ballonkatheter nach einem der vorhergehenden Ansprüche, **dadurch gekennzeichnet, dass** in dem Lumen (7a) des Katheterschafts (7) ein Innenschaft (8) zur Aufnahme eines Führungsdrahts angeordnet ist, wobei die beiden Leiter (50, 60) auf einer Außenseite (8a) des Innenschafts (8) im Lumen (7a) des Katheterschafts (7) verlaufen, oder dass die beiden Leiter (50, 60) in dem Lumen (7a) des Katheterschafts verlaufen, wobei der Katheterschaft (7a) zumindest ein benachbartes weiteres Lumen (8b) zur Aufnahme eines Führungsdrahts aufweist.

**14.** Verfahren zum Messen eines Ballondurchmessers (D) unter Verwendung eines Ballonkatheters (1) nach einem der vorhergehenden Ansprüche, aufweisend die Schritte:

　　- Expandieren des Ballons (3) in der radialen Richtung (R) durch Einleiten eines fluiden und elektrisch leitfähigen Inflationsmediums (M) in den Balloninnenraum (3a), so dass zwischen den beiden Elektroden (5, 6) eine Zelle (V) des Inflationsmediums (M) gebildet wird, und
　　- Messen eines elektrischen Widerstands der Zelle (V) des Inflationsmediums (M) mittels der Elektroden (5, 6) und automatisches Berechnen des Durchmessers (D) des Ballons (3) anhand des gemessenen elektrischen Widerstands.

**15.** Verfahren zur Herstellung einer Elektrode (5, 6) für einen Ballonkatheter (1), insbesondere für einen Ballonkatheter (6) nach einem der vorhergehenden Ansprüche, aufweisend die Schritte:

　　- Bereitstellen eines Drahts (50, 60) aus einer Formgedächtnislegierung, wobei der Draht (50, 60) einen distalen und einen damit verbundenen proximalen Abschnitt (51, 52; 61, 62) aufweist,
　　- Umformen und Wärmebehandeln des distalen Abschnitts (51, 61) derart, dass der distale Abschnitt (51, 61) bei einer Temperatur oberhalb einer Umwandlungstemperatur der Formgedächtnislegierung einen gekrümmten und/oder abgewinkelten Verlauf gegenüber dem proximalen Abschnitt (52, 62) aufweist,
　　- Ausbilden einer plattenförmigen Elektrode (5, 6) an einem distalen Ende (51a, 61a) des distalen Abschnitts (51, 62), und
　　- Ummanteln des distalen und des proximalen Abschnitts (51, 52; 61, 62) des Drahts (50, 60) mit einer elektrischen Isolierung (53, 63), wobei die Elektrode (5, 6) freigelassen wird.

**FIG. 1**

**FIG. 2A**

**FIG. 2B**

**FIG. 2C**

**FIG. 2D**

FIG. 3

54
8
50
53
8a
52
51b
51
51a
5
H
R
R
A
64
60
63
62
61b
61
61a
2

FIG. 4A

FIG. 4B

FIG. 4C

FIG. 5A

FIG. 5B

FIG. 5C

Europäisches
Patentamt

European
Patent Office

Office européen
des brevets

## EUROPÄISCHER RECHERCHENBERICHT

Nummer der Anmeldung

EP 19 18 5653

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 904 967 A1 (ST JUDE MEDICAL CARDIOLOGY DIV [US]) 12. August 2015 (2015-08-12) | 1-7, 11-14 | INV. A61M25/10 |
| Y | * Abbildungen 3, 5, 9A, 9B * * Absatz [0038] * * Absatz [0039] * * Absatz [0041] * ----- | 8-10 | |
| X | WO 2016/090175 A1 (METAVENTION INC [US]) 9. Juni 2016 (2016-06-09) | 15 | |
| Y | * Abbildung 18 * * Absatz [0059] * * Absatz [0293] * ----- | 8-10 | |
| A | US 5 397 308 A (ELLIS LOUIS [US] ET AL) 14. März 1995 (1995-03-14) * Spalte 2, Zeile 11 - Zeile 37 * ----- | 1-15 | |
| A | US 2013/123694 A1 (SUBRAMANIYAN RAGHAVAN [IN] ET AL) 16. Mai 2013 (2013-05-16) * Ansprüche 1-34 * ----- | 1-15 | |

RECHERCHIERTE SACHGEBIETE (IPC)

A61B
A61M

Der vorliegende Recherchenbericht wurde für alle Patentansprüche erstellt

| Recherchenort | Abschlußdatum der Recherche | Prüfer |
|---|---|---|
| Berlin | 9. Januar 2020 | Przykutta, Andreas |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes
    Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 19 18 5653

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-01-2020

| Im Recherchenbericht angeführtes Patentdokument | | Datum der Veröffentlichung | Mitglied(er) der Patentfamilie | | Datum der Veröffentlichung |
|---|---|---|---|---|---|
| EP 2904967 | A1 | 12-08-2015 | EP | 2904967 A1 | 12-08-2015 |
| | | | US | 2015223729 A1 | 13-08-2015 |
| | | | US | 2018078177 A1 | 22-03-2018 |
| WO 2016090175 | A1 | 09-06-2016 | AU | 2015358385 A1 | 13-07-2017 |
| | | | CA | 2969129 A1 | 09-06-2016 |
| | | | EP | 3226795 A1 | 11-10-2017 |
| | | | JP | 2017536187 A | 07-12-2017 |
| | | | US | 2019069949 A1 | 07-03-2019 |
| | | | WO | 2016090175 A1 | 09-06-2016 |
| US 5397308 | A | 14-03-1995 | KEINE | | |
| US 2013123694 | A1 | 16-05-2013 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 97142871 A
- US 5827273 A